# EUROPÄISCHE PATENTSCHRIFT

(11) **EP 1 231 201 B1**
(45) Veröffentlichungstag und Bekanntmachung des Hinweises auf die Patenterteilung: **20.04.2011**
(21) Anmeldenummer: 02002119.2
(22) Anmeldetag: 29.01.2002
(51) Int. Cl.: C07C 57/04

(54) **Verfahren zur Herstellung von Anhydriden ungesättiger Carbonsäuren**
Process for the preparation of anhydrides of unsaturated carboxylic acids
Procédé pour la préparation d'anhydrides d'acides carboxyliques insaturés

(30) Priorität: 09.02.2001 DE 10106352
(43) Veröffentlichungstag der Anmeldung: 14.08.2002
(73) Patentinhaber: Evonik Röhm GmbH, 64293 Darmstadt (DE)
(72) Erfinder: Schmitt, Bardo, 55120 Mainz (DE); Knebel, Joachim, 64665 Alsbach (DE); Klesse, Wolfgang, 55127 Mainz (DE); Wittkowski, Andrea, 64823 Gross-Umstadt (DE); Laux, Bededikt, 55234 Monternheim (DE)

(56) Entgegenhaltungen:
- DE-A- 3 644 222
- FR-A- 863 141
- FR-A- 2 592 040
- GB-A- 612 790
- US-A- 2 319 070
- BECKER AND VOGEL: "Influence of the metal ions Cr3+,Fe3+,Ni2+, and Cu2+ on the stability and oxygen consumption of acrylic- and methacrylic acid", CHEMICAL ENGINEERING AND TECHNOLOGY, vol. 25, no. 5, 1 January 2002 (2002-01-01), pages 547-552, WEINHEIM ISSN: 0930-7516

## Beschreibung

### GEBIET DER ERFINDUNG

Die Erfindung beschreibt einen Prozess zur Darstellung von ungesättigten Carbonsäureanhydriden, insbesondere die Reaktion einer ungesättigten Carbonsäure mit einem niedermolekularen aliphatischen Carbonsäureanhydrid.

### STAND DER TECHNIK

DE 35 10 035 beschreibt ein Verfahren zur kontinuierlichen Herstellung von Carbonsäureanhydriden durch die Umsetzung von Acetanhydrid mit einer Carbonsäure. Dieses Verfahren der Umanhydrisierung ist allgemein bekannt. WO 95/32940 beschreibt ein Verfahren zur Synthese von ungesättigten Carbonsäureanhydriden, wie beispielsweise Acrylsäureanhydrid oder Methacrylsäureanhydrid durch eine Reaktion eines aromatischen Säurechlorids, wie beispielsweise Benzoylchlorid, mit den Anionen der ungesättigten Carbonsäure. Dieses Verfahren hat aus industrieller Sicht betrachtet den Nachteil, dass pro Mol gebildeten Anhydrids ein Mol Natriumchlorid als zu entsorgender Abfallstoff anfällt.

FR 2592040 beschreibt die Synthese von Methacrylsäureanhydrid durch die Reaktion von Essigsäureanhydrid mit Methacrylsäure in Abwesenheit eines Katalysators. Es ist notwendig, die Reaktionsmischung durch Anwesenheit eines Polymerisationsinhibitors zu stabilisieren.

Es gibt eine Vielzahl an Möglichkeiten, Methacrylsäureanhydrid (MASA) herzustellen. So erhält man MASA aus Methacrylsäure und dem entsprechenden Säurechlorid nach WO 9532940, US 4,874,558 oder SU 228016. Ein anderer Syntheseweg ist der als Phasentransferkatalyse beschriebene mit den oben genannten Produkten in Lab. Chim. Org., CNRS, Rennes-Beaulieu, Fr. Tetrahedron (1988), 44(9), 2471-6. DE 35 44 765 beschreibt die Carbonylierung von Methacrylsäureestern unter Metallkatalyse bei hoher Temperatur und hohem Druck. Über Dehydrierung der Methacrylsäure lässt sich ebenfalls MASA mit den Katalysatoren AcOC(CN)₂Me (JP 49034655) oder (CN)₂ plus Ni(OAc)₂ herstellen. Eine einfache Synthese ist die Reaktion von MAS und Essigsäureanhydrid (EA). In DE 35 10 035 ist sie als kontinuierlicher Prozess unter Säurekatalyse beschrieben. Ganz ohne Katalysator kommt FR 2592040 als Batchversuch aus.

DE 36 44 222 beschreibt die Synthese von Carbonsäureanhydriden (unter anderem wird auch ASA nebenbei im Text erwähnt) in Gegenwart von Metallionen der Gruppe Mn, Fe, Co, Ni und Mg, die unter anderem auch als Acetat vorliegen. Diese Redoxmetalle neigen stark zu Veränderungen in der Oxidationsstufe, was im Falle der Synthese von ungesättigten Carbonsäuren sich nachteilig auf den Reaktionsverlauf auswirkt und zu Polymerisation führt. Deshalb wird in dieser Erfindung auf andere, nicht die Polymerisation fördernde Metallsalze zurückgegriffen. Das Verfahren hebt sich deshalb von DE 36 44 222 ab, da andere Metallacetate verwendet werden. Weiterhin wird mit Unterdruck gearbeitet, was mit einer geringeren Temperaturbelastung verbunden ist und es wird das Zulaufverfahren benutzt, wodurch die Raum-Zeit-Ausbeute gesteigert wird. Aus der DE 36 44 222 ist ein Verfahren bekannt, bei dem Carbonsäureanhydride hergestellt werden, in Gegenwart eines Metalliones ausgewählt aus der Gruppe Mn, Fe, Co, Ni oder Mg. Die Reaktion wird im Temperaturbereich zwischen 10 °C bis hin zum Siedepunkt des niederen aliphatischen Säureanhydrids durchgeführt. Es werden hier allerdings bevorzugt aromatische Carbonsäuren- und aromatische Polycarbonsäuren und niedere aliphatische Carbonsäureanhydride umgesetzt.

### AUFGABE

Es bestand also die Aufgabe, die bekannten Verfahren zur Herstellung von Methacrylsäureanhydrid dahingehend zu verbessern, dass einmal der Halogenanfall vermieden wird und dass zweitens durch die Verwendung eines entsprechenden Katalysators die Reaktionszeit und die Raumzeitausbeute der Umanhydrisierungsreaktion gesteigert wird.

### LÖSUNG

Die Aufgabe wird durch die Reaktion der Methacrylsäure mit Essigsäureanhydrid in Gegenwart eines Katalysators und Inhibitors gelöst. Als Katalysator dient ein Metallsalz einer organischen Verbindung. Als Metall kommen die Metalle Cr, Zn, Cu, Ca, Na, Ti, Zr, Hf, La in Frage. Die organische Verbindung besitzt mindestens eine Carboxylgruppe.

Unter den organischen Verbindungen, die mindestens eine Carboxylgruppe besitzen, werden Carbonsäuresalze niederer aliphatischer Carbonsäuren, wie beispielsweise Acetate, Propionate, Butyrate, Laurate, Salicylate, usw. und β-Diketone wie beispielsweise Acetylacetonate (2,4-Pentandionate), 3,5-Heptandionate und Benzoylacetonate oder β-Ketocarbonsäuren wie beispielsweise Acetoacetate oder β-Ketocarbonsäuresalze wie beispielsweise Ethylacetoacetate oder Dicarbonsäuren wie beispielsweise Oxalsäure und Malonsäure verstanden. Besonders bevorzugt sind Metallsalze in Form der Acetate und Acetylacetonate. Der Katalysator kann sowohl für die Durchführung der Reaktion im Batchreaktor eingesetzt werden wie auch in Reaktionen, die nach dem Zulaufverfahren oder kontinuierlichen Verfahren durchgeführt werden. Führt man die Reaktion als Zulaufverfahren durch, wird Methacrylsäure und die Hälfte des Essigsäureanhydrids im Reaktor vorgelegt, die andere Hälfte des Essigsäureanhydrids wird im Verlaufe der Reaktion zudosiert. Das molare Verhältnis von Essigsäureanhydrid zur Methacrylsäure variiert von 0,5 bis 1, bevorzugt ist das Verhältnis 0,55 bis 0,65, besonders bevorzugt ist das Verhältnis von 0,58 bis 0,62. Die Verwendung des Katalysators verkürzt die Reaktionszeit stark.

Da der Katalysator als Feststoff oder gelöst in der Reaktionslösung verbleibt, kann er auch leicht abgetrennt werden. Das Methacrylsäureanhydrid kann durch Destillation leicht abgetrennt werden, die teilweise stark gefärbte Reaktionslösung hat keine Auswirkung auf die Farbe des erhaltenden Produktes.

Als Stabilisatoren können alle gängigen Inhibitoren, u. a. Hydrochinon, Hydrochinonmonomethylether, Topanol O, Topanol A, Phenothiazin, Irganox 1010 (eingetr. WZ der Ciba AG) und N,N'-Diphenyl-p-phenylendiamin sowie deren Mischungen verwendet werden.

### Allgemeine Versuchsvorschrift

Methacrylsäure, Essigsäureanhydrid und die Stabilisatoren sowie der Katalysator werden im Reaktionskolben vorgelegt. Die Apparatur wird auf 95 mbar evakuiert und der Ansatz auf Siedetemperatur erhitzt. Im Anschluß wird das essigsäurehaltige Destillat abgezogen und die zweite Portion Essigsäureanhydrid kontinuierlich zugetropft.

Bleibt die Kopftemperatur konstant, wird der Druck langsam reduziert. Als Endwert wird ein Druck von ca. 2-20 m bar erreicht. Nach beendeter Reaktion wird der Ansatz auf Raumtemperatur abgekühlt und der Sumpf per GC analysiert. Das Rohprodukt hat einen Gehalt an MASA von 97-98 %. Durch fraktionierte Destillation kann eine Reinheit von weit über 99 % erzielt werden.

### Ansatz:

| | |
|---|---|
| 322,8 g (3,75 mol) | Methacrylsäure |
| 114,9 g (1,13 mol) | Essigsäureanhydrid vorgelegt |
| 114,9 g (1,13 mol) | Essigsäureanhydrid zugetropft |
| 1,61g (0,5 % bez. auf MAS) | Katalysator s. Tabelle |
| 1000 mg (3100 ppm bez. auf MAS) | Phenothiazin |
| 200 mg (620 ppm bez. auf MAS) | Hydrochinon |

### Vergleichsversuch

Der Vergleichsversuch wird analog, nur ohne Katalysatorzugabe durchgeführt und wird in der Tabelle als Standardansatz bezeichnet. Hier dauert die Reaktion länger als mit Katalysator. Außerdem ist die Reinheit des Rohprodukts und die Ausbeute geringer.

**Tabelle Versuchsergebnisse**

| Beispiel | Zusammensetzung Sumpf | | | Bemerkungen / Katalysator |
|---|---|---|---|---|
| | Reaktionszeit h | MASA FI% | Ausbeute % | |
| Vergleichsversuch | 5,5 | 96,66 | 61,9 | **Standardansatz, ohne Katalysator** |
| 1 | 4,25 | 98,35 | 78,2 | Chromacetat |
| 2 | 3,5 | 97,1 | 69,2 | Zinkacetat |
| Vergleichsversuch | 3,5 | 97,51 | 70,8 | Kupferacetat*Monohydrat |
| 4 | 3,5 | 97,14 | 73,9 | Calciumacetat*Monohydrat |
| 5 | 4,5 | 96,65 | 72,2 | Natriumacetat |
| 6 | 4,25 | 98,24 | 76,8 | Zirkonacetylacetonat |
| 7 | 4,5 | 96,41 | 65 | Chromacetylacetonat |
| 8 | 4 | 97,4 | 74,5 | Lanthanacetylacetonat-hydrat |
| 9 | 4 | 98,56 | 73,5 | Hafniumacetylacetonat |
| 10 | 4,25 | 98,6 | 74,9 | Titanacetylacetonat |

## Patentansprüche

1. Verfahren zur Herstellung von Methacrylsäureanhydrid durch Umsetzung von Methacrylsäure und Essigsäureanhydrid in Gegenwart eines Stabilisators und eines Katalysators in Form eines Metallsalzes einer organischen Verbindung mit mindestens einer Carboxylgruppe oder einer Diketongruppe, wobei als Kation im Metallsalz Cr, Zn, Ca, Zr, Ti, Na, La, Hf einzeln oder als Mischsalz verwendet werden.

2. Verfahren nach Anspruch 1,
**dadurch gekennzeichnet, dass**
als organische Verbindungen Carbonsäuren, Dicarbonsäuren, beta-Ketocarbonsäuren, beta-Diketone einzeln oder in Mischungen verwendet werden.

3. Verfahren nach Anspruch 1,
**dadurch gekennzeichnet, dass**
als Katalysatoren Chromacetat, Zirkonacetylacetonat oder Titanacetylacetonat einzeln oder in Mischungen verwendet werden.

## Claims

1. Process for preparing methacrylic anhydride by reacting methacrylic acid with acetic anhydride in the presence of a stabilizer and a catalyst in the form of a metal salt of an organic compound having at least one carboxyl group or one diketone group, where the cation used in the metal salt is Cr, Zn, Ca, Zr, Ti, Na, La, Hf alone or a mixed salt.

2. Process according to Claim 1, **characterized in that** the organic compounds used are carboxylic acids, dicarboxylic acids, beta-ketocarboxylic acids or beta-diketones, alone or in mixtures.

3. Process according to Claim 1, **characterized in that** the catalysts used are chromium acetate, zirconium acetylacetonate or titanium acetylacetonate, alone or in mixtures.

## Revendications

1. Procédé pour la préparation d'anhydride méthacrylique par mise en réaction d'acide méthacrylique et d'anhydride acétique en présence d'un stabilisant et d'un catalyseur sous forme d'un sel métallique d'un composé organique comportant au moins un groupe carboxy ou un groupe dicétone, en utilisant comme cation dans le sel métallique Cr, Zn, Ca, Zr, Ti, Na, La, Hf, seuls ou sous forme de sel mixte.

2. Procédé selon la revendication 1, **caractérisé en ce qu'**on utilise comme composés organiques des acides carboxyliques, des acides dicarboxyliques, des acides bêta-cétocarboxyliques, des bêta-dicétones, seuls ou en mélanges.

3. Procédé selon la revendication 1, **caractérisé en ce qu'**on utilise comme catalyseurs l'acétate de chrome, l'acétylacétonate de zirconium ou l'acétylacétonate de titane, seuls ou en mélanges.
